# EUROPEAN PATENT APPLICATION

(11) **EP 3 348 241 A1**
(43) Date of publication of application: **18.07.2018**
(21) Application number: 17828832.0
(22) Date of filing: 21.07.2017
(51) Int. Cl.: A61F 5/56

(54) **SLEEPING RESPIRATORY TRACT ADJUSTMENT DISTANCE GENERATING METHOD, SOFTWARE, SYSTEM AND RECORDING MEDIUM**

(30) Priority: 30.08.2016 CN 201610770717
(71) Applicant: Soteria Biotech Co., Ltd., Taipei 10452 (TW)
(72) Inventor: HSIAO, Hung-Ta, Taipei 10452 (TW); LO, Ing-Hsien, Taipei 10452 (TW)
(74) Representative: Lang, Christian
(86) International application number: PCT/CN2017/000463
(87) International publication number: WO 2018/040453

(57) **Abstract**

The present invention provides a method for generating an adjusted distance of a sleep respiratory tract, and a software, an apparatus system and a computer readable recording medium thereof. The method includes the steps of: reading a first medical image; establishing a first respiratory tract model; performing a first breath simulation; reading a second medical image; establishing a second respiratory tract model; performing a second breath simulation; and calculating an adjusted distance. With the implementation of the present invention, a correct generated distance of the adjusted distance of the sleep respiratory tract is provided according to the established models and the results of the breath simulation when the patient's respiratory tract tomography is read twice respectively. Besides, the adjusted distance of the sleep respiratory tract can be used as a reference for adjusting a sleep respiratory brace and for doctors in a lower jaw corrective surgery.

## Description

### BACKGROUND OF THE INVENTION

### 1. Technical Field

The present invention relates to a method for generating an adjusted distance of a sleep respiratory tract, and a software, an apparatus system and a computer readable recording medium thereof. More particular, the present invention relates to a method for generating an adjusted distance of a sleep respiratory tract, and a software, an apparatus system and a computer readable recording medium thereof that are provided for patients with sleep apnea syndrome and for simulating a corrective parameters.

### 2. Description of Related Art

With the progress of life and technology, there have been many means or methods for preventing sleep-disordered respiration, wherein the snoring treatment is the most fundamental way to prevent sleep-disordered respiration or sleep apnea.

The apnea hypopnea index (AHI) of many patients with sleep apnea syndrome is up to 50 times per night. The treatment methods recommended by doctors are anti-snoring surgeries, anti-snoring pillows, respirators, thermoplastic anti-snoring braces or customized anti-snoring braces. Considering that there is no need for a surgery and using an expensive (about 80,000 to 90,000 TWD) and inconvenient respirator while ensuring a relatively stable effect, a customized lower jaw advanced anti-snoring brace (Mandibular Advanced Device) is a solution that is simple to produce and use and is inexpensive without invasive treatment.

In general, by using the customized anti-snoring braces, accurate occlusion can be achieved and the lower jaw is trimmed artificially to fix the lower jaw in an advanced way, and thus the mouth may assume an underbite state. The dentists can periodically adjust the advanced distance of the lower jaw according to the extent of posterior collapse of tongue and surrounding tissues. With a relatively stable wearing effect and more effective pull, the customized anti-snoring braces pull and fix the position of lower jaw, so that tongue and surrounding tissues do not interfere with respiration and sleep.

The way to artificially trim the lower jaw by the customized anti-snoring braces is done by trial and error, and that is assuming an effective value firstly and then adjusting according to the applying result. The problems are the high frequency of adjustments and the patients have to endure the discomfort in using stage before achieving therapeutic value.

In addition, in an anti-snoring surgery, a common practice is that the lower jaw of a patient is advanced or extended by a surgeon to avoid that the respiratory tract is squeezed, and thus to achieve the effect of anti-snoring. During the surgery, in order to achieve effective treatment, how far should the surgeon advance or extend the lower jaw? Since the distance was determined by the personal experience of the surgeon, the risk and failure rate of the surgery is higher.

In view of this, how to develop an innovative invention provided for patients with sleep apnea syndrome, wherein desired expansion distance of lower jaw has been mastered before the customized anti-snoring braces is produced, or desired corrective expansion distance has been mastered before the doctor performs the operation by image analysis and simulation analysis techniques to avoid failure by scientific and accurate way, will become a great boon for patients with sleep apnea syndrome.

### SUMMARY OF THE INVENTION

The present invention provides a method for generating an adjusted distance of a sleep respiratory tract, and a software, an apparatus system and a computer readable recording medium thereof, which reads the patient's respiratory tract tomography twice respectively, and thus a correct corrective expansion distance is provided according to established models and the results of the breath simulation. Besides, the correct corrective expansion distance can be used as a reference for adjusting the patient's sleep respiratory brace quickly and correctly, or for doctors to use in a lower jaw corrective surgery.

The present invention provides a method for generating an adjusted distance of a sleep respiratory tract, which comprises the steps of: reading a first medical image, wherein the first medical image is a first respiratory tract tomography of a patient read by a computation module; establishing a first respiratory tract model, wherein the first respiratory tract model is established by the computation module according to the first respiratory tract tomography; performing a first breath simulation, wherein the first breath simulation is performed by the computation module according to the first respiratory tract model, and data of a first respiratory pressure distribution, a first magnitude of a pressure differential, and a first jet expansion angle are obtained; reading a second medical image, wherein the second medical image is a second respiratory tract tomography of the patient read by the computation module when an occlusal structure is bitten by the patient; establishing a second respiratory tract model, wherein the second respiratory tract model is established by the computation module according to the second respiratory tract tomography; performing a second breath simulation, wherein the second breath simulation is performed by the computation module according to the second respiratory tract model, and data of a second respiratory pressure distribution, a second magnitude of a pressure differential, and a second jet expansion angle are obtained; and calculating an adjusted distance, wherein the adjusted distance is a corrective expansion distance which is calculated by the computation module according to the data of the first respiratory pressure distribution, the first magnitude of the pressure differential, the first jet expansion angle, the second respiratory pressure distribution, the second magnitude of the pressure differential, and the second jet expansion angle.

The present invention also provides a software for performing the above-mentioned method for generating a sleep respiratory brace.

The present invention further provides an apparatus system storing internally the above-mentioned software, wherein the apparatus system is used to perform the software.

The present invention also further provides a computer readable recording medium that records a program for causing a computer to execute the above-mentioned software when the program is loaded by the computer.

With the implementation of the present invention, at least the following progressive effects can be achieved:
1. The manufacturing process is simple without using additional complex instruments, and it can save the cost of arrangement and using;
2. The correct respiratory tract model of patients can be obtained directly without performing trial and error of experiment;
3. It is easy to verify or confirm, and it can significantly reduce errors in adjusting the sleep respiratory brace and save expenses and time costs;
4. The accurate corrective expansion distance of the sleep respiratory brace can be obtained; and
5. The present invention can provide for doctors as a reference in the lower jaw corrective surgery.

In order that the technology contents of the present invention will be readily understood and implemented by those skilled in the relevant art, and related objects and advantages of the present invention may be readily understood by those skilled in the relevant art according to the disclosure, scope of the appended claims and accompanying drawings of the description, the detailed features and advantages of the present invention will be described in detail in the embodiments. The foregoing description is merely a general description of the technical solution of the present invention. In order to better understand the technical means of the invention to implement them according to the description, and to make the above-mentioned and other objects, features and advantages of the present invention more obvious, the following preferred embodiments will be made with reference to the accompanying drawings, which are described in detail below.

### BRIEF DESCRIPTION OF THE SEVERAL VIEWS OF THE DRAWINGS

FIG. 1A is a schematic view of various parts of the upper respiratory tract defined medically.
FIG. 1B is a flowchart showing the steps of a method for generating an adjusted distance of a sleep respiratory tract according to an embodiment of the present invention.
FIG. 2 is a side view of a first respiratory tract model according to an embodiment of the present invention;
FIG. 3A is a front view of a first respiratory pressure simulation according to an embodiment of the embodiment;
FIG. 3B is a side view of a first respiratory pressure simulation according to an embodiment of the embodiment;
FIG. 4 is a diagram of a first jet expansion angle simulation according to an embodiment of the embodiment;
FIG. 5 is a side view of a second respiratory tract model according to an embodiment of the present invention;
FIG. 6 is a perspective view of an adjustable occlusal splint according to an embodiment of the present invention;
FIG. 7 is a diagram of the overlapped FIGs. 2 and 5 to show and compare the differences before and after the correction of respiratory tract;
FIG. 8A is a front view of a second respiratory pressure simulation according to an embodiment of the present invention;
FIG. 8B is a side view of a second respiratory pressure simulation according to an embodiment of the present invention; and
FIG. 9 is a diagram of a second jet expansion angle simulation according to an embodiment of the present invention.

### LIST OF REFERENCE NUMERAL

| | |
|---|---|
| R100: upper respiratory tract | R10: nasal |
| R20: velopharynx | R30: oropharynx |
| R40: hypopharynx | R50: trachea |
| S100: method for generating an adjusted distance of a sleep respiratory tract | |
| S10: step of reading a first medical image | |
| S20: step of establishing a first respiratory tract model | |
| S30: step of performing a first breath simulation | |
| S40: step of reading a second medical image | |
| S50: step of establishing a second respiratory tract model | |
| S60: step of performing a second breath simulation | |
| S70: step of calculating an adjusted distance | |
| S80: step of adjusting the sleep respiratory brace | |
| 10: adjustable occlusal splint | |
| 11: bite portion | 12: adjusting portion |
| D1: first vertical reference distance | D2: second vertical reference distance |

### DETAILED DESCRIPTION OF THE EMBODIMENTS OF THE INVENTION

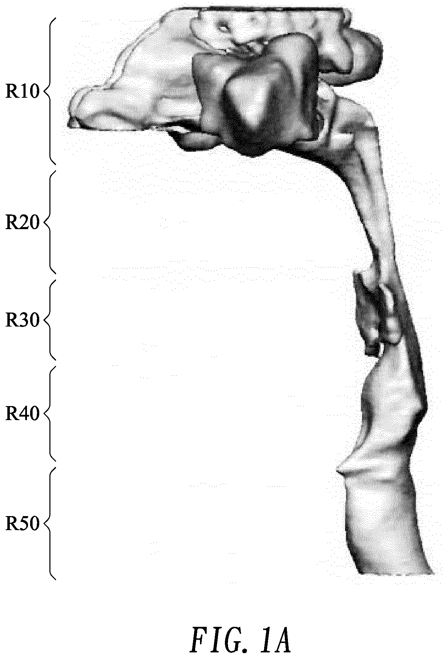

As shown in FIG. 1A, according to the medical definition, relevant upper respiratory tract R100 mainly includes a nasal R10, a velopharynx R20, a oropharynx R30, a hypopharynx R40, and a trachea R50. In embodiments of the present invention, the described respiratory tract refers to all parts from the velopharynx R10, the oropharynx R20, the hypopharynx R30 to the trachea R40.

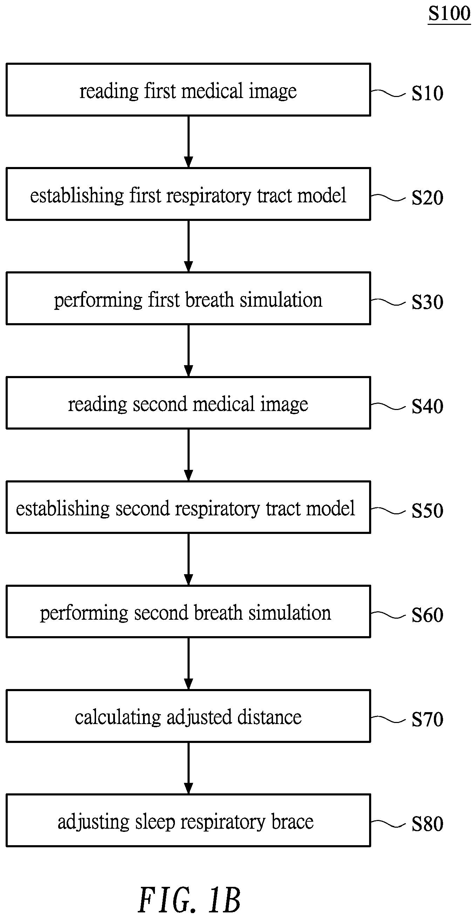

In reference to FIG. 1B, an embodiment of the present invention provides a method S100 for generating an adjusted distance of a sleep respiratory tract, which may be executed in a computer system. The method S100 for generating an adjusted distance of a sleep respiratory tract includes the steps of: reading a first medical image (step S10); establishing a first respiratory tract model (step S20); performing a first breath simulation (step S30); reading a second medical image (step S40); establishing a second respiratory tract model (step S50); performing a second breath simulation (step S60); and calculating an adjusted distance (step S70).

As shown in FIG. 1B, the step S10 of reading the first medical image is reading a first respiratory tract tomography of a patient by a computation module. The first respiratory tract tomography can be images of computed tomography (CT) or cone beam computed tomography (CBCT).

In order to read the first respiratory tract tomography, the images can be read by the computation module directly by being connected to medical CT or CBCT device in a wireless or wired manner. The computation module can also read the image data stored in advance and generated by the CT or CBCT device to obtain the first respiratory tract tomography.

The computation module used herein can be a computer system, a microprocessor system, a personal computer, a notebook computer, or an intelligent device comprising at least one computation or application software.

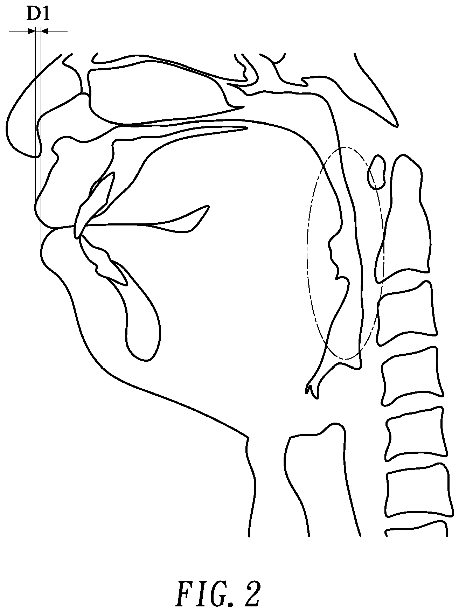

As shown in FIGs. 1B and 2, the step S20 of establishing a first respiratory tract model is establishing the first respiratory tract model corresponding to the patient's respiratory tract by the computation module according to the first respiratory tract tomography, and the first respiratory tract model can be a three-dimensional (3D) respiratory tract model of the patient.

In one embodiment, the computation module can correctly obtain the position and shape of the respiratory tract from the first respiratory tract tomography by comparing gray level or values, and thus to establish the first respiratory tract model of the respiratory tract of the patient.

Also as shown in FIG. 1B, the step S30 of performing a first breath simulation is performing the first breath simulation by the computation module according to the first respiratory tract model, and data of a first respiratory pressure distribution, a first magnitude of a pressure differential and a first jet expansion angle, and associated data information about breath state of the patient such as breath flow rate at the narrowest area are obtained. In terms of the apnea hypopnea index (AHI), an AHI of being less than 5 times per hour is normal.

The definition of the jet expansion angle refers to an angle formed between two jet half-width lines along the long axial direction of the respiratory tract. The jet half-width refers to a position where the respiratory tract extends on both sides to half of the maximum jet velocity at any position in horizontal axis direction with maximum jet velocity being a central point. At this point, the distance between the central point of the maximum jet velocity and the position of half of the maximum jet velocity is referred to the jet half-width.

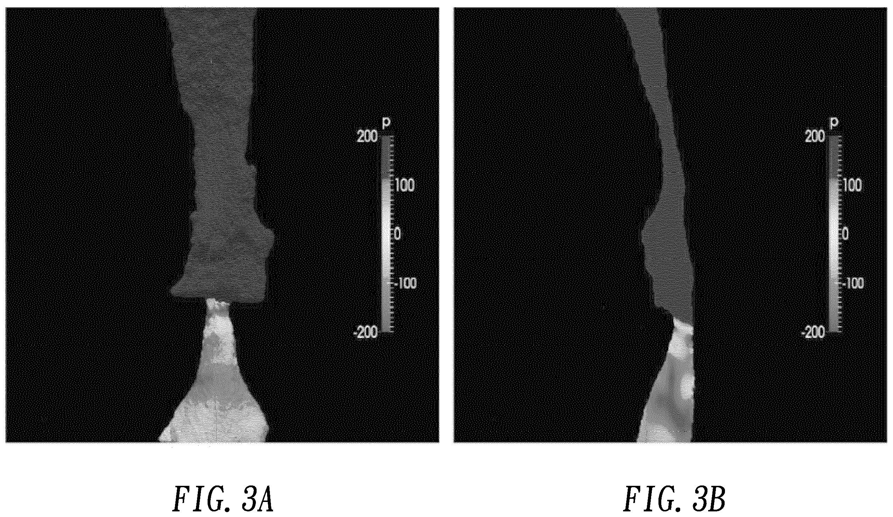

Also as shown in FIGs. 3A and 3B, the scale index of pressure in figures is from -200 to 200, and the unit of pressure P is Pa (N/m²). From the front view and side view of simulation results of the respiratory tract, we can learn that, when a subject has not accepted the correction, the first vertical reference distance between the upper jaw and the lower jaw is D1. At this point, since the upper respiratory tract is not expanded but in an oppressed state, the simulation in the upper respiratory tract shows the dark state, which indicates the existence of uneven pressure distribution in the respiratory tract and a very large pressure difference.

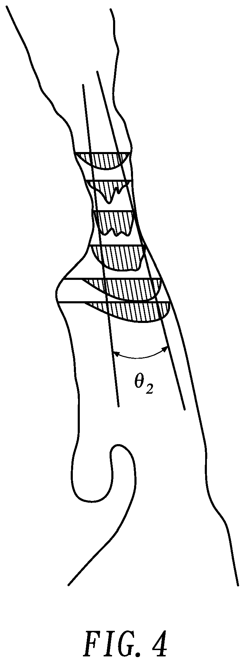

As shown in FIG. 4, after the simulation of the first jet expansion angle of the respiratory tract is performed, we can learn that without the correction, the respiratory tract shows a larger jet expansion angle with 0̸₁=8.3°, and has different width.

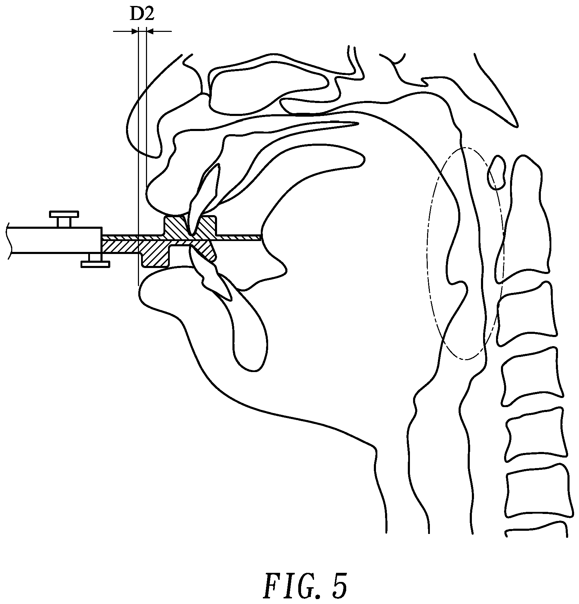

Next, in reference to FIGs. 1B and 5, the step S40 of reading the second medical image is obtaining a second respiratory tract tomography of the patient read by the computation module when an occlusal structure is bitten by the patient. The occlusal structure used herein can be a brace, an occlusal splint, an adjustable brace, or an adjustable occlusal splint 10 with higher elasticity.

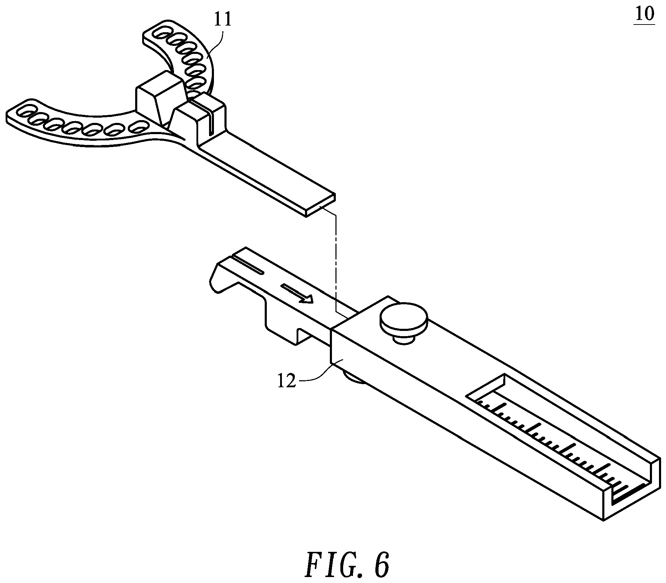

As shown in FIG. 6, the adjustable occlusal splint 10 can include a bite portion 11 and an adjusting portion 12, wherein the bite portion 11 is bitten in the mouth of the patient, and the adjusting portion 12 is used for adjusting a position of the bite portion 11 in the mouth of the patient.

The second respiratory tract tomography can also be the images of computed tomography (CT) or cone beam computed tomography (CBCT). The second respiratory tract tomography and the first respiratory tract tomography should be the same kind images of CT or CBCT, which can obtain the most accurate results of the method S100 for generating the adjusted distance of the sleep respiratory tract.

As shown in FIG. 1B, the step S50 of establishing a second respiratory tract model is establishing the second respiratory tract model corresponding to the patient's respiratory tract by the computation module according to the read second respiratory tract tomography, and the second respiratory tract model can be a three-dimensional (3D) respiratory tract model of the patient.

In one embodiment, the computation module can also correctly obtain the position and shape of the respiratory tract from the second respiratory tract tomography by comparing gray level or values, and thus to establish the second respiratory tract model of the respiratory tract of the patient.

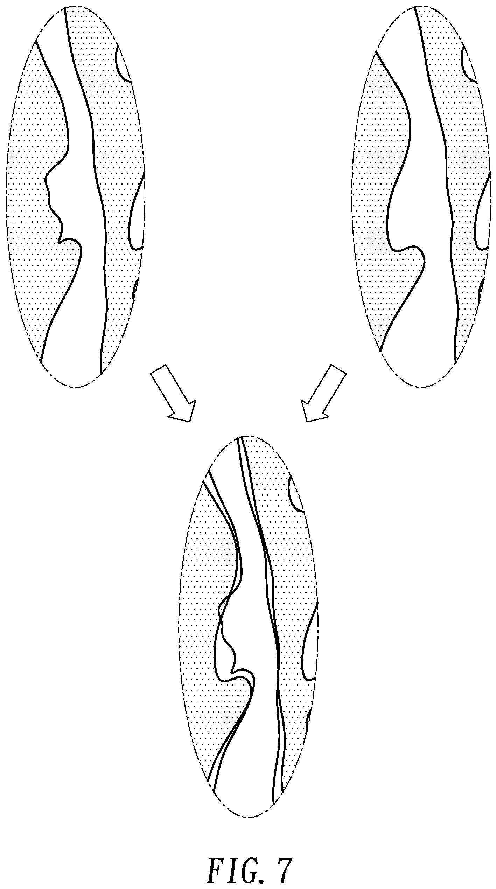

As shown in FIG. 7, after FIGs. 2 and 5 are overlapped, it can be clearly observed that comparing with the respiratory tract of the first respiratory tract tomography, the respiratory tract of the second respiratory tract tomography expands a spatial volume to a level.

Also as shown in FIG. 1B, the step S60 of performing a second breath simulation is performing the breath simulation by the computation module according to the second respiratory tract model, and data of a second respiratory pressure distribution, a second magnitude of a pressure differential and a second jet expansion angle, and associated data information about breath state when the occlusal structure is bitten by the patient are obtained.

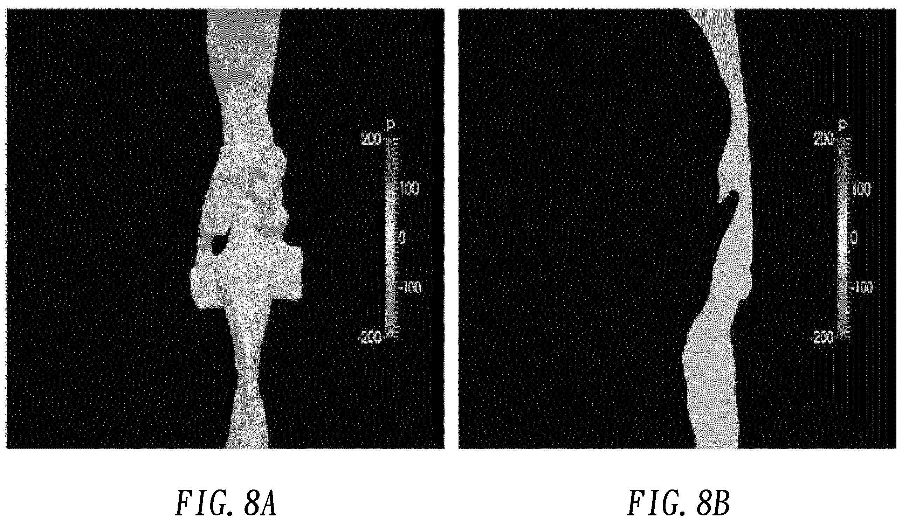

Also as shown in FIGs. 8A and 8B, the scale index of pressure in figures is from -200 to 200 and the unit of pressure P is Pa (N/m²). From the front view and side view of simulation results of the respiratory tract, we can learn that, when a subject has accepted the correction after wearing the adjustable occlusal splint 10, the second vertical reference distance between the upper jaw and the lower jaw is D2, and the second vertical reference distance D2 is greater than the first vertical reference distance D1. At this point, since the upper respiratory tract is expanded due to outward move of the lower jaw, the simulation in the upper respiratory tract shows the light state, which indicates an even pressure distribution in the respiratory tract and a reduced and similar pressure.

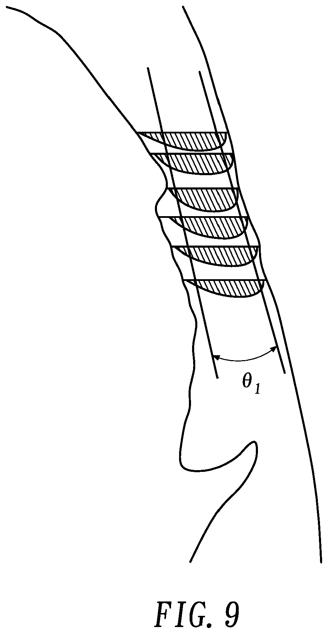

As shown in FIG. 9, after the simulation of the second jet expansion angle of the respiratory tract is performed, we can learn that without the correction, the respiratory tract shows a smaller jet expansion angle with 0̸₂=4.2°, and has similar width.

In reference to FIG. 1B, the step S70 of calculating an adjusted distance is calculating a corrective expansion distance by the computation module according to the data of the first respiratory pressure distribution, the first magnitude of the pressure differential, the first jet expansion angle, the second respiratory pressure distribution, the second magnitude of the pressure differential, and the second jet expansion angle, wherein the corrective expansion distance is the adjusted distance.

The corrective expansion distance can be obtained by calculating the data of the first respiratory pressure distribution, the first magnitude of the pressure differential, the first jet expansion angle, the second respiratory pressure distribution, the second magnitude of the pressure differential, and the second jet expansion angle by the computation module using interpolation method, heterodyne method, average calculation method, or database comparison-bioinformatics.

Next, in reference to FIG. 1B again, the step S80 of adjusting a sleep respiratory brace is adjusting the sleep respiratory brace of the patient by the computation module according to the calculated and obtained corrective expansion distance.

The embodiment can further include the step S80 of adjusting the sleep respiratory brace, wherein the sleep respiratory brace is adjusted by using the above-mentioned corrective expansion distance, and the lower jaw part of the sleep respiratory brace is advanced or expanded, which can be done manually or by an adjusting device.

Furthermore, if the patient is assessed to be suitable for orthodontic surgery, the corrective expansion distance can be provided to the surgeon to use as a reference during the surgical procedure to determine how far the surgeon should advance or extend the lower jaw of the patient. Thus, the success rate of surgery can be improved more accurately.

In this way, with the implementation of the method S100 for generating the adjusted distance of the sleep respiratory tract, since the patient can wear a sleep respiratory brace which is accurately adjusted or the doctor can master the advanced or expanded distance of the lower jaw, the respiration and sleep will not be interfered by improper position of the tongue and surrounding tissues, and snoring or dangerous sleep apnea can be prevented more effectively. During the implementation of the embodiment, the above-mentioned method S100 for generating the adjusted distance of the sleep respiratory tract, in particular, can further be performed by a software.

In addition, the software for performing the method S100 for generating an adjusted distance of the sleep respiratory tract can further be stored internally in an apparatus system for executing the above-mentioned software, for example, a computer apparatus is capable of executing the above-mentioned software.

In addition, a program can further be recorded in a computer readable recording medium and the program can cause a computer to execute the above-mentioned software performing the method S100 for generating the adjusted distance of the sleep respiratory tract when the computer loads the program.

The above description is only the preferred embodiments of the present invention, and is not intended to limit the present invention in any form. Although the invention has been disclosed as above in the preferred embodiments, they are not intended to limit the invention. A person skilled in the relevant art will recognize that equivalent embodiment modified and varied as equivalent changes disclosed above can be used without parting from the scope of the technical solution of the present invention. All the simple modification, equivalent changes and modifications of the above embodiments according to the material contents of the invention shall be within the scope of the technical solution of the present invention.

## Claims

1. A method for generating an adjusted distance of a sleep respiratory tract, comprising the steps of:
reading a first medical image, wherein the first medical image is a first respiratory tract tomography of a patient read by a computation module;
establishing a first respiratory tract model, wherein the first respiratory tract model is established by the computation module according to the first respiratory tract tomography;
performing a first breath simulation, wherein the first breath simulation is performed by the computation module according to the first respiratory tract model, and data of a first respiratory pressure distribution, a first magnitude of a pressure differential, and a first jet expansion angle are obtained;
reading a second medical image, wherein the second medical image is a second respiratory tract tomography of the patient read by the computation module when an occlusal structure is bitten by the patient;
establishing a second respiratory tract model, wherein the second respiratory tract model is established by the computation module according to the second respiratory tract tomography;
performing a second breath simulation, wherein the second breath simulation is performed by the computation module according to the second respiratory tract model, and data of a second respiratory pressure distribution, a second magnitude of a pressure differential, and a second jet expansion angle are obtained; and
calculating an adjusted distance, wherein the adjusted distance is a corrective expansion distance which is calculated by the computation module according to the data of the first respiratory pressure distribution, the first magnitude of the pressure differential, the first jet expansion angle, the second respiratory pressure distribution, the second magnitude of the pressure differential, and the second jet expansion angle.

2. The method of claim 1, wherein the first respiratory tract tomography and the second respiratory tract tomography are the images of computed tomography.

3. The method of claim 1, wherein the first respiratory tract tomography and the second respiratory tract tomography are the images of cone beam computed tomography.

4. The method of claim 1, wherein the step of establishing the first respiratory tract model is establishing a three-dimensional respiratory tract model of the patient.

5. The method of claim 1, wherein the step of establishing the second respiratory tract model is establishing a three-dimensional respiratory tract model of the patient.

6. The method of claim 1, wherein the corrective expansion distance is obtained by calculating with the computation module using interpolation method, heterodyne method, average calculation method, or database comparison-bioinformatics.

7. The method of claim 1, wherein the computation module is a computer system, a microprocessor system, a personal computer, a notebook computer, or an intelligent device comprising at least one computation or application software.

8. The method of claim 1, further comprising a step of adjusting a sleep respiratory brace, wherein a lower jaw part of the sleep respiratory brace is advanced or expanded according to the corrective expansion distance.

9. The method of claim 1, wherein the occlusal structure is a brace, an occlusal splint, an adjustable brace, or an adjustable occlusal splint.

10. The method of claim 1, wherein the step of adjusting the sleep respiratory brace is performed by an adjusting device.

11. A software for performing a method for generating a sleep respiratory brace of any of claims 1 to 6.

12. An apparatus system internally storing a software of claim 11, wherein the apparatus system is used to perform the software.

13. A computer readable recording medium that records a program for causing a computer to execute a software of claim 11 when the program is loaded by the computer.
